# FASCICULE DE BREVET EUROPEEN

(11) **EP 3 852 845 B1**
(45) Date de publication et mention de la délivrance du brevet: **08.11.2023**
(21) Numéro de dépôt: 19795253.4
(22) Date de dépôt: 19.09.2019
(51) Int. Cl.: A61M 5/32, A61M 5/20

(54) **AUTOINJECTEUR**
AUTOINJEKTOR
AUTOINJECTOR

(30) Priorité: 20.09.2018 FR 1858533
(43) Date de publication de la demande: 28.07.2021
(73) Titulaire: APTAR France SAS, 27110 Le Neubourg (FR)
(72) Inventeur: FABIEN, David, 22130 Corseul (FR)
(74) Mandataire: CAPRI
(86) Numéro de dépôt international: PCT/FR2019/052192
(87) Numéro de publication internationale: WO 2020/058639

(56) Documents cités:
- EP-A1- 2 438 946
- EP-A1- 2 705 862
- US-A- 5 865 804
- US-A- 6 149 626
- US-A1- 2003 105 430

## Description

La présente invention concerne un autoinjecteur.

Les autoinjecteurs sont bien connus dans l'état de la technique. Ces dispositifs ont principalement pour but de réaliser une injection automatique du contenu d'une seringue ou d'une cartouche à l'intérieur du corps d'un patient. Divers systèmes existent pour rendre automatique l'injection du produit fluide contenu dans la seringue. Les autoinjecteurs sont des dispositifs relativement complexes qui doivent répondre à un certain nombre d'exigences de contraintes pour être fiables. La robustesse du dispositif, sa maniabilité, et sa facilité d'utilisation pour l'utilisateur sont également des éléments importants. Par ailleurs, le coût de fabrication et d'assemblage est également un facteur dont il faut tenir compte.

Il existe de nombreux autoinjecteurs sur le marché, qui peuvent présenter des inconvénients.

Ainsi, les autoinjecteurs utilisent généralement un ou plusieurs ressort(s), maintenus sous charges avant le déclenchement, pour effectuer l'injection, et parfois aussi le piquage. Ces ressorts peuvent être de tous types, notamment à traction, compression, torsion, spirale ou autres. L'effort généré par le ressort disponible pour pousser le piston lors de l'expulsion du produit fluide est dans la plupart des cas directement lié aux caractéristiques force/longueur ou autres paramètres dimensionnels du ressort, tel que l'angle pour un ressort de torsion. Le ressort d'injection exerce alors une force maximale au début, cette force diminuant progressivement au cours de la phase d'injection. Ceci peut notamment présenter des problèmes selon la viscosité du fluide à distribuer. De plus, le ressort doit exprimer une puissance importante au début de l'injection, pour garantir que la phase d'injection arrivera à son terme, ce qui peut rendre douloureux le début de la phase d'injection.

En réalité, la force appliquée par le ou les ressorts de l'autoinjecteur sur la tige de piston devrait idéalement pouvoir être modulée au cours de la course d'actionnement afin de s'adapter aux différentes étapes rencontrées par le piston lors de l'expulsion. Ainsi, lors l'accostage du piston par la tige de piston, il faut éviter un choc trop violent afin d'éviter d'endommager voire casser le réservoir, en particulier la collerette dans le cas d'une seringue. D'un autre côté, il peut exister un phénomène de collage du piston dans le réservoir du au stockage, ce qui requiert un pic d'effort pour initier le déplacement du piston. Par ailleurs, certains produits fluides peuvent nécessiter une certaine variation d'effort lors de leur expulsion en raison de leurs caractéristiques. On peut ainsi citer les fluides non newtoniens qui ont la particularité d'avoir une viscosité qui dépend de la vitesse des molécules. Ainsi, pendant les premiers millimètres de la course du piston, un pic d'effort est nécessaire afin d'initier le déplacement du piston. De plus, certains fluides très visqueux nécessitent d'appliquer un effort important sur le piston, typiquement de l'ordre de 100 à 200 Newtons. Or, l'effort nécessaire pour déclencher cette force d'expulsion très important doit rester acceptable pour l'utilisateur, typiquement de l'ordre de 4 à 8 Newtons, et être répétable et maitrisé.

D'autre part, un point critique des autoinjecteurs actuels généralement à usage unique est que leur fonction principale, à savoir la distribution du produit fluide, ne peut être testée au préalable, avant utilisation par l'utilisateur final.

Un autre problème se pose avec certains autoinjecteurs, notamment ceux qui sont à usage unique et donc jetable après usage. Or un tel dispositif complexe comporte de nombreuses pièces, et notamment des pièces métalliques, tels que les ressorts, ce qui pose de problème de recyclage. En effet, il est potentiellement difficile de recycler le métal d'un autoinjecteur car il est classé en déchets d'activités de soins à risques infectieux (DASRI).

Les documents EP2438946, US6149626, EP2705862, US2003105430 et US5865804 décrivent des dispositifs de l'état de la technique.

La présente invention a pour but de fournir un autoinjecteur qui ne reproduit pas les inconvénients susmentionnés.

La présente invention a aussi pour but de fournir un autoinjecteur avec un profil de force prédéterminé pendant toute la phase d'injection.

La présente invention a également pour but de fournir un autoinjecteur dont la majeure partie est réutilisable, et dont la partie jetable après chaque actionnement ne contient pas de métal.

La présente invention a aussi pour but de fournir un autoinjecteur que le fabriquant peut tester avant de le mettre dans les mains de l'utilisateur.

La présente invention a aussi pour but de fournir un autoinjecteur qui soit fiable et sûr d'utilisation, qui permette de garantir la distribution de la totalité du produit fluide à l'endroit souhaité, et qui soit simple et peu coûteux à fabriquer et à assembler.

La présente invention a donc pour objet un autoinjecteur tel que décrit dans la revendication 1. Des modes de réalisation avantageux sont décrits dans les revendications dépendantes.

Ces caractéristiques et avantages et d'autres apparaîtront plus clairement au cours de la description détaillée suivante, faite en référence aux dessins joints, donnés à titre d'exemples non limitatifs, et sur lesquels :
La figure 1 est une vue schématique de côté en perspective éclatée des trois modules d'un autoinjecteur, selon un mode de réalisation avantageux,
La figure 2 est une vue schématique de côté en perspective selon un autre angle de vue du dispositif de la figure 1, avec le module interface assemblé sur le module moteur et avant assemblage du module réservoir,
La figure 3 est une vue similaire à celle de la figure 2, après assemblage du module réservoir,
La figure 4 est une vue similaire à celle de la figure 3, avant retrait du capuchon de protection d'aiguille,
La figure 5 est une vue similaire à celle de la figure 4, après retrait du capuchon de protection d'aiguille,
La figure 6 est une vue similaire à celle de la figure 5, après déverrouillage du module interface,
La figure 7 est une vue similaire à celle de la figure 6, en début d'actionnement, avant piquage de l'aiguille,
La figure 8 est une vue similaire à celle de la figure 7, en cours d'actionnement, après piquage de l'aiguille et avant injection,
La figure 9 est une vue similaire à celle de la figure 8, après injection et avant retrait du site d'injection,
La figure 10 est une vue similaire à celle de la figure 9, après retrait du site d'injection et avant réarmement du module moteur,
La figure 11 est une vue similaire à celle de la figure 10, après réarmement du module moteur et avant déverrouillage du module réservoir,
La figure 12 est une vue similaire à celle de la figure 11, après déverrouillage du module réservoir,
La figure 13 est une vue schématique en section transversale d'un autoinjecteur selon un mode de réalisation avantageux, avant assemblage du module réservoir,
La figure 14 est une vue similaire à celle de la figure 13, en début d'assemblage du module réservoir,
La figure 15 est une vue similaire à celle de la figure 14, en fin d'assemblage du module réservoir,
La figure 16 est une vue similaire à celle de la figure 15, en cours d'actionnement, après piquage de l'aiguille et avant injection,
La figure 17 est une vue schématique de détail en section transversale d'une serrure d'injection selon un mode de réalisation avantageux, en position verrouillée,
La figure 18 est une vue schématique de détail de la serrure de la figure 17,
La figure 19 est une vue similaire à celle de la figure 17, en position déverrouillée,
La figure 20 est une vue schématique de côté en perspective d'un autoinjecteur, selon un mode de réalisation avantageux, après injection,
La figure 21 est une vue schématique partielle en perspective découpée illustrant des moyens de réarmement de l'autoinjecteur de la figure 20,
La figure 22 est une vue schématique en perspective d'un organe de réarmement utilisable avec les moyens de réarmement de la figure 21,
La figure 23 est une vue schématique de côté en perspective d'un autoinjecteur, selon un autre mode de réalisation avantageux, après piquage,
La figure 24 est une vue schématique partielle découpée du dispositif de la figure 23,
La figure 25 illustre de manière schématique, en section transversale, un exemple avantageux de came permettant de prédéterminer le profil de force durant la phase d'injection,
Les figures 26 à 28 sont des vues similaires à celle de la figure 25, qui illustrent le dispositif successivement en début et en cours d'injection, et
La figure 29 illustre la courbe d'effort lors des différentes phases d'injection, selon un mode de réalisation avantageux.

L'autoinjecteur va être décrit ci-après en référence à plusieurs modes de réalisation avantageux de celui-ci. Il est toutefois à noter que les autoinjecteurs, qui sont des appareils complexes, comprennent plusieurs modules pour réaliser plusieurs fonctions. Ces divers modules peuvent être utilisés séparément et indépendamment les uns des autres, sans être nécessairement combinés aux autres modules, et pourraient notamment être utilisés dans des autoinjecteurs de forme différente de celle représentée sur les dessins. De plus, il est à noter que les dessins sont des vues schématiques qui ne représentent pas forcément la forme exacte des composants d'un autoinjecteur, et ne sont pas forcément à l'échelle, notamment dans des buts de clarté. Par ailleurs, les dessins ne représentent pas forcément tous les éléments constitutifs d'un autoinjecteur, mais seulement les éléments nécessaires au fonctionnement de la présente invention. Ainsi, divers éléments et modules supplémentaires et/ou complémentaires pourraient être associés à l'autoinjecteur représenté sur les figures.

Dans les modes de réalisation représentés sur les dessins, l'autoinjecteur comporte trois modules ou parties distinctes : un module réservoir 100, un module moteur 200 et un module interface 300.

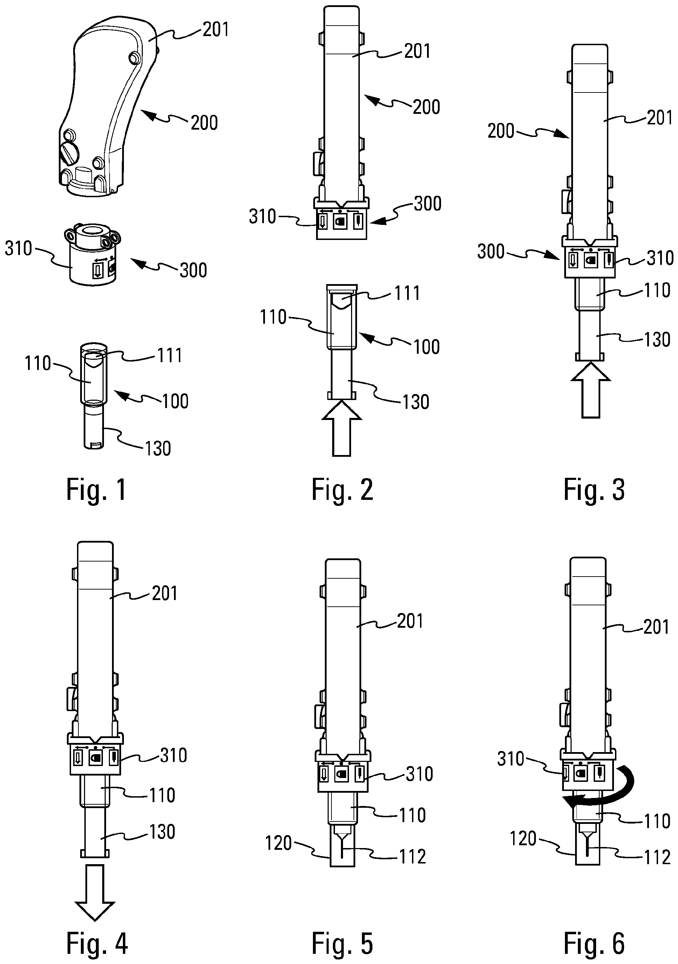

La figure 1 montre les trois modules séparés et les figures 2 à 12 montrent l'assemblage du module réservoir plein, l'actionnement du module moteur et l'extraction du module réservoir vide après l'actionnement.

Le module réservoir 100 comporte un réservoir 110 qui contient du produit fluide à injecter, un piston 111 et une aiguille 112. Le piston 111 est adapté à se déplacer dans ledit réservoir 110 pour injecter le produit fluide à travers ladite aiguille 112. Plus généralement, il est entendu que le terme « réservoir » dans la présente description englobe tout type de contenant associé à une aiguille, tel qu'une seringue ou une cartouche. L'aiguille 112 peut être fixée de manière permanente audit réservoir 110, ou en variante, l'aiguille 112 peut être assemblée sur le réservoir 110 avant l'actionnement de l'autoinjecteur.

Dans les modes de réalisation décrits sur les figures, le réservoir 110 est une cartouche, de préférence réalisée en verre.

Le module réservoir 100 comporte le moins de pièces possible, car jetable, et de préférence pas de ressort ou autre pièce métallique.

Le module réservoir 100 comporte un manchon actionneur 120 dont la surface d'extrémité axiale est en contact avec le site à injecter et comporte une ouverture permettant le passage de l'aiguille 112 lors de la phase de piquage. Après actionnement, le manchon actionneur 120 recouvre l'aiguille 112 du réservoir pour éviter tout risque de piqure avec ladite aiguille. Le manchon actionneur 120 est adapté à coulisser par rapport audit réservoir 110 entre une position déployée, dans laquelle il est disposé autour de ladite aiguille 112 et une position actionnée, dans laquelle il est déplacé axialement par rapport au réservoir 110 pour exposer l'aiguille 112.

Lorsque le réservoir 110 comporte une aiguille 112, le module réservoir 100 comporte un capuchon 130 de protection d'aiguille qui peut être retiré avant utilisation de l'autoinjecteur, avantageusement au moyen d'un organe de retrait 135 approprié.

Le module moteur 200 contient les moyens d'injection et est de préférence réutilisable. Pour être actionné, il doit de préférence être associé au module interface 300 car l'injection est avantageusement déclenchée par celui-ci. Ce module moteur 200 permet de faire varier de manière prédéterminée la force d'actionnement Fa exercée sur la tige de piston au cours de la phase d'injection, comme cela sera expliqué plus en détails ci-après.

Le module moteur 200 comporte un corps 201, qui est avantageusement formé de deux coques creuses fixées l'une à l'autre. Ce corps contient une tige de piston 210 qui lors de l'actionnement va venir au contact du piston 111 du réservoir 110 et le déplacer axialement dans le réservoir 110 pour injecter le produit fluide. Cette tige de piston 210 est avantageusement guidée axialement dans un profil de guidage approprié du corps 201, tel que par exemple un rail, une rainure ou une pièce de centrage traversée par ladite tige de piston 210.

Le corps 201 du module moteur 200 contient également les moyens d'injection et de réarmement qui seront décrits plus en détails ci-après.

Le module interface 300 permet de relier le module moteur 200 au module réservoir 100. Il est également de préférence réutilisable. Ce module interface 300 permet d'une part l'insertion du module réservoir 100. Lorsque le réservoir 110 est un cartouche, comme dans les exemples des figures, le module interface 300 permet aussi le vissage du sous-ensemble aiguille et l'extraction du capuchon 130 de protection d'aiguille. Avantageusement, ce vissage et/ou cette extraction du capuchon n'est (ne sont) possible(s) que lorsque le module réservoir 100 est assemblé sur le module interface 300. Ce dernier permet aussi le déplacement du manchon actionneur 120 en début d'actionnement pour réaliser le piquage et déclencher la phase d'injection, et son verrouillage en position de sécurité de l'aiguille après l'injection. Enfin, il permet aussi l'extraction du module réservoir 100 vide pour être remplacé par un nouveau module réservoir 100 plein.

Le module interface 300 comporte une bague de sélection 310 montée rotative par rapport au module moteur 200 et des éléments pivotants 320.

Dans la position de la figure 2, le module interface 300 est assemblé sur le module moteur 200. Avant assemblage du module réservoir 100, la bague de sélection 310 est en position de verrouillage, dans laquelle l'insertion du module réservoir 100 est possible et l'actionnement du module moteur 200 est impossible.

L'utilisateur insert un module réservoir 100 non utilisé dans le module interface 300, comme illustré sur les figures 2 et 3. Avantageusement, il est impossible d'insérer un module réservoir utilisé, par exemple grâce à un système de détrompeur. Eventuellement, à la fin de l'assemblage du module réservoir 100, un clic sonore se fait entendre.

L'utilisateur retire ensuite le capuchon 130 de protection d'aiguille, comme illustré sur les figures 4 et 5. Eventuellement, lorsque la conception du réservoir 100 ne comporte pas d'aiguille, le sous-ensemble aiguille est d'abord assemblé sur le module réservoir, de préférence après l'assemblage dudit module réservoir 100 sur le module interface 300.

La bague de sélection 310 est ensuite déverrouillée vers une position d'injection, avantageusement via une rotation exercée par l'utilisateur, comme illustré sur la figure 6.

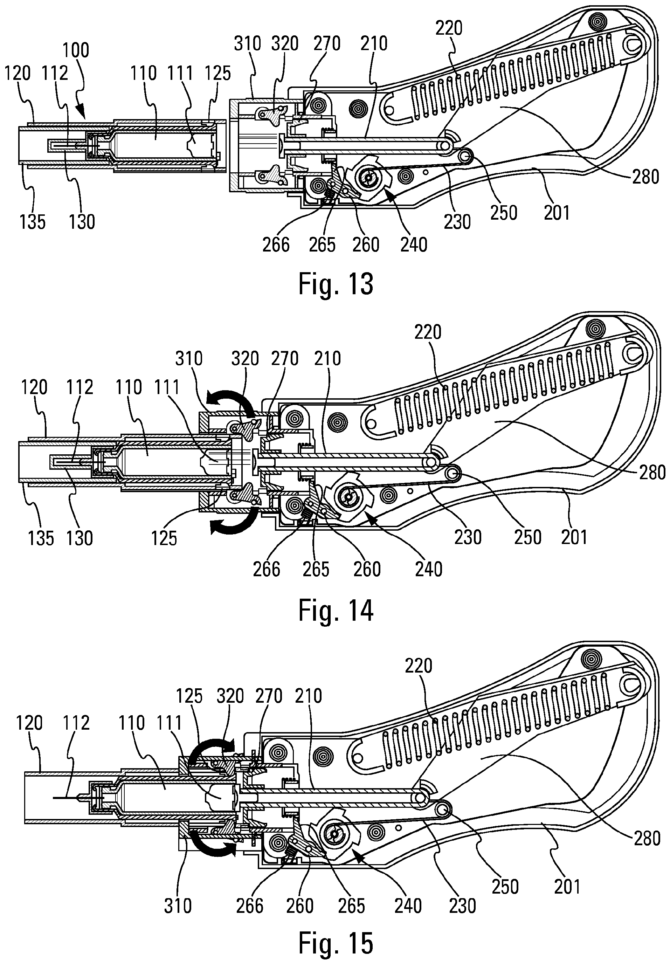

Les figures 13 à 15 illustrent l'assemblage du module réservoir 100 sur le module interface 300. Les éléments pivotants 320 du module interface 300 permettent le verrouillage du module réservoir 100 sur le module interface 300.

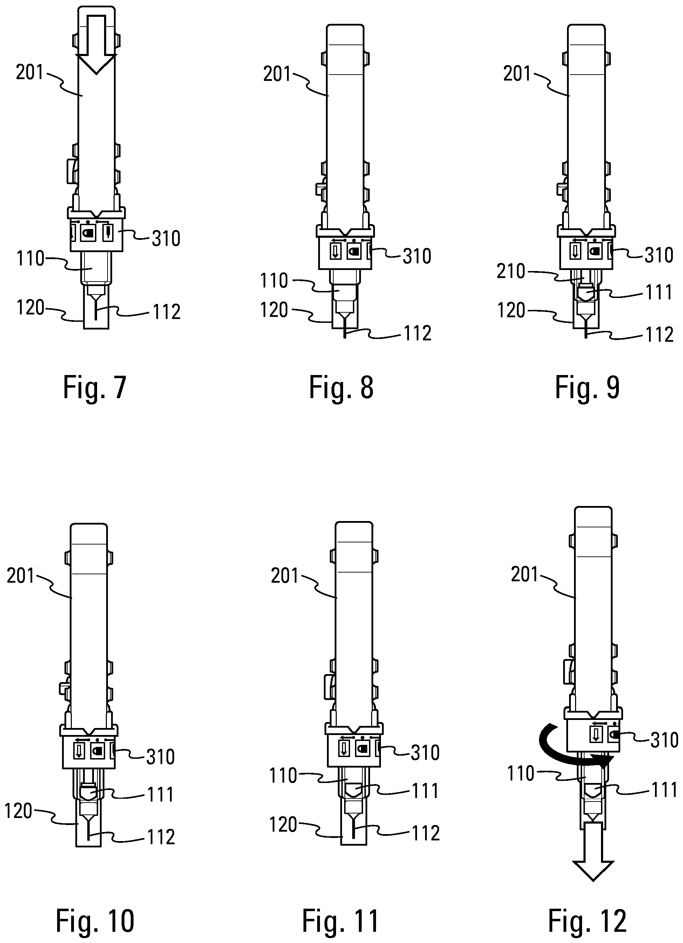

Les figures 7 à 10 illustrent le piquage et l'injection.

En début d'actionnement, l'utilisateur appuie l'autoinjecteur sur le site à injecter, ce qui provoque le coulissement axial du manchon actionneur 120 dans le module réservoir 100, entrainant ainsi l'aiguille 112 à piquer ledit site à injecter. Avantageusement, l'utilisateur doit appuyer avec une force supérieure à un seuil prédéterminable formé par un point dur prévu pour empêcher tout coulissement accidentel dudit manchon actionneur 120.

Pendant la phase de piquage, la bague de sélection 310 tourne automatiquement vers une position de sécurité indiquant que le module réservoir 100 se mettra en sécurité aiguille après l'injection lors du déploiement du manchon actionneur 120.

En fin de piquage, le manchon actionneur 120 coopère avec la serrure du module moteur pour déclencher la phase d'injection, et le produit fluide contenu dans le réservoir 110 est expulsé par le piston 111 poussé par la tige de piston 210 qui est pilotée en force par un ressort d'actionnement 220.

Lorsque l'injection est terminée, l'utilisateur relâche la pression de l'autoinjecteur sur la peau, et le manchon actionneur 120 se déploie et se verrouille en position de sécurité.

L'utilisateur peut alors réarmer le module moteur 200, comme cela sera décrit plus en détails ci-après.

Pour éjecter le module réservoir 100 vide, l'utilisateur tourne la bague de sélection 310 en position « éjection », et le module réservoir 110 est déverrouillé et translaté axialement de quelques millimètres. L'utilisateur finit ensuite de retirer manuellement le module réservoir 100 utilisé.

Lorsqu'elle est relâchée, la bague de sélection 310 revient automatiquement en position initiale verrouillée, et elle ne peut alors plus être déplacée avant l'assemblage d'un nouveau module réservoir 100 plein dans le module interface 300.

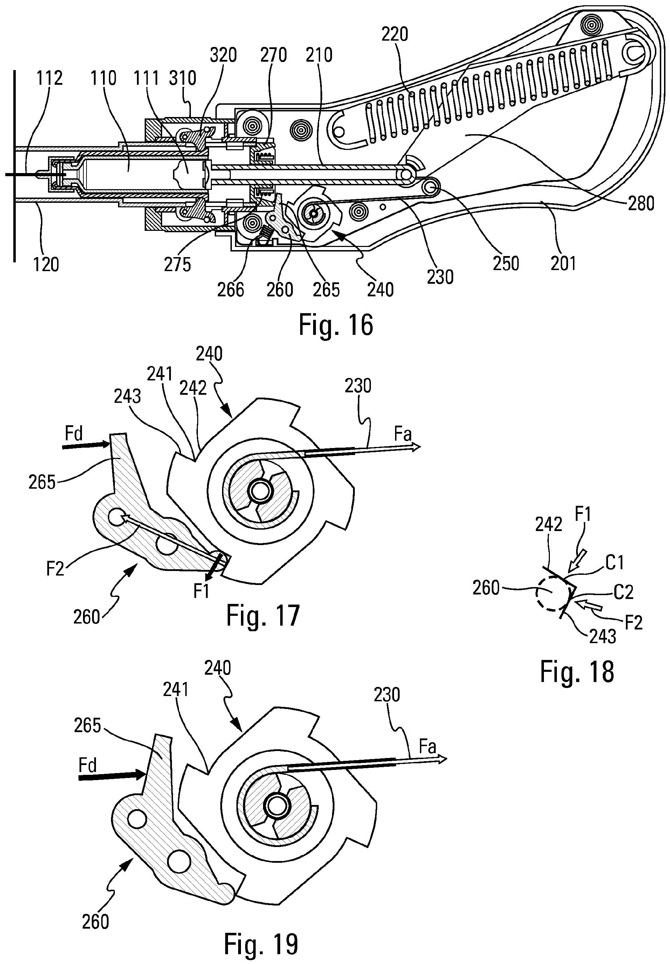

Les figures 17 à 19 illustrent plus particulièrement la serrure d'injection.

Dans ce mode de réalisation, la tige de piston 210 est reliée par un fil ou courroie 230 (ou similaire) à une roue d'enroulement dentée 240, avantageusement via une roue de renvoi 250 formant poulie. Le ressort d'injection 220 exerce une force d'actionnement Fa sur la tige de piston 210, qui est transmise à la roue d'enroulement 240 par ledit fil 230.

Un organe de verrouillage 260 coopère avec les dents 241 de ladite roue d'enroulement dentée 240, ledit organe de verrouillage 260 étant mobile, notamment pivotant, entre une position de blocage, visible sur la figure 17, et une position d'actionnement, visible sur la figure 19.

Avantageusement, un élément élastique 266, par exemple un ressort, sollicite ledit organe de verrouillage 260 vers sa position de blocage.

Avantageusement, l'organe de verrouillage 260, en position de blocage, coopère avec une dent 241 de la roue d'enroulement 240 avec deux points de contacts C1 et C2 disposés sur deux surfaces 242 et 243 non parallèles, comme visible sur la figure 18. Ceci génère donc deux forces orientées différemment F1 et F2.

L'organe de verrouillage 260 comporte une extension 265, adaptée à coopérer avec le manchon actionneur 120 lorsque celui-ci atteint sa position d'actionnement, c'est-à-dire une fois que l'aiguille 112 a piqué le site à injecter. De préférence, une bague intermédiaire 270 est prévue entre le manchon actionneur 120 et l'organe de verrouillage 260. Ainsi, après piquage, le manchon actionneur 120 pousse sur ladite bague intermédiaire 270 avec une force de déverrouillage Fd, et ladite bague intermédiaire 270 transmet cette force Fd à ladite extension 265 de l'organe de verrouillage 260, ce qui fait pivoter celui-ci vers sa position d'actionnement. La roue d'enroulement 240 n'est alors plus bloquée en rotation, et le ressort d'injection 220 peut déplacer la tige de piston 210 pour réaliser la phase d'injection.

Pendant cette phase d'injection, le fil 230 se déroule donc de ladite roue d'enroulement 240.

Lors de l'injection, la bague intermédiaire 270 est avantageusement sollicitée élastiquement en contact avec le manchon actionneur 120, par exemple par un ressort 275, de sorte que le manchon actionneur 120 est sollicité vers sa position de sécurité après l'injection.

Cette géométrie de serrure permet de maitriser l'effort nécessaire à l'utilisateur pour déverrouiller la serrure et donc déclencher l'injection. En particulier, elle permet de garantir un effort minimum pour l'utilisateur afin de déverrouiller un gros effort d'injection.

Par exemple, une force de déverrouillage Fd de 5N peut être suffisante pour déverrouiller un ressort d'injection 220 capable d'exercer une force d'actionnement Fa d'environ 100N sur la tige de piston 210.

De plus, la serrure forme un système « engageant », c'est à dire que l'orientation des efforts permet d'avoir un système auto bloquant. Ainsi, une augmentation de la force d'actionnement Fa a pour effet de renforcer le verrouillage de la serrure.

Le dispositif comporte des moyens de maitrise d'effort, afin de faire varier de manière prédéterminée la force exercée par le ressort d'injection 220 sur le piston 111 du réservoir 110 pendant toute la phase d'injection.

Pour ce faire, on prévoit l'utilisation d'un levier 280, qui est relié à la tige de piston 210, au ressort d'injection 220 et à une came 290, de préférence solidaire du corps 201 du module moteur 200.

En variante, on pourrait imaginer que la came soit formée sur le levier et qu'elle coopère avec un point fixe solidaire du corps.

Le ressort d'injection 220 est avantageusement fixé d'un côté au corps 201 et de l'autre côté audit levier 280, dont une extrémité est pivotante sur la tige de piston 210 et dont l'autre extrémité se déplace le long de ladite came 290.

Pendant l'injection, le levier 280 effectue de préférence à la fois une rotation autour d'un point de la tige de piston 210 et un mouvement de translation par rapport au corps 201, ces deux mouvements étant liés et réalisés dans un même plan.

En fonction du profil de ladite came 290, on peut prédéterminer la force d'actionnement Fa qui sera appliquée par le ressort d'injection 220 sur la tige de piston 210 pendant toute la phase d'injection.

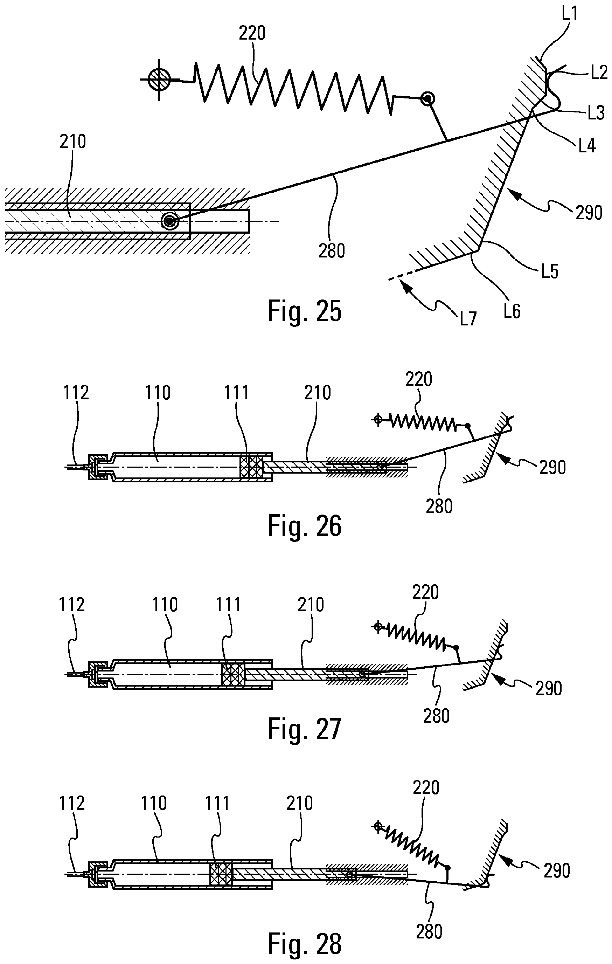

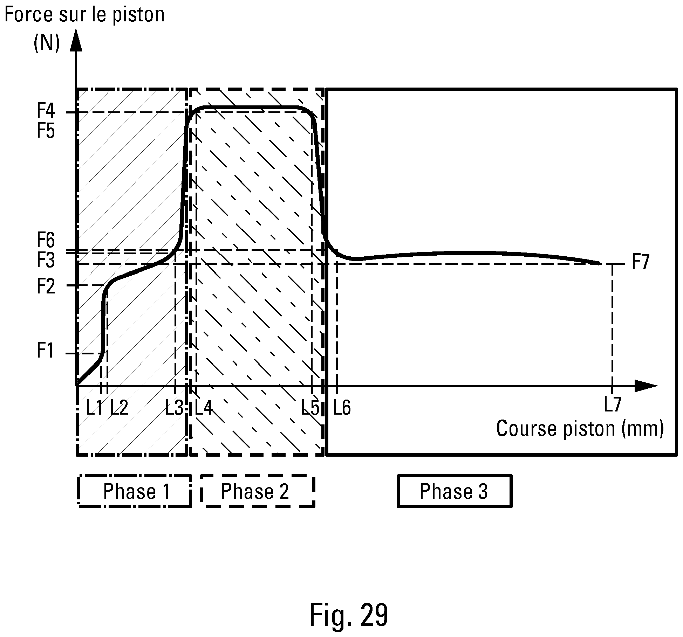

La figure 29 montre une courbe de cette force Fa dans l'exemple de la came représentée sur les figures 25 à 28.

Dans cet exemple, il y a trois phases distinctes :
Phase 1 : accostage & compression piston
Course: 4 mm
   - Accostage (section avant L1):
      Course: 2mm
      Force Fa: croissante de 0 à 18N
   - Compression stopper (section L2-L3):
      Course: 2mm
      Force Fa: croissante de 57 à 72N
Phase 2: mise en mouvement du fluide (section L4-L5):
   Course: 6mm
   Force Fa: 150N constante
Phase 3: Injection du fluide (section après L6):
   Course: 12mm
   Force Fa: 60N constante

Bien entendu, ces valeurs chiffrées ne sont pas limitatives, et ne représente qu'un exemple particulier.

Chaque section correspond à une forme spécifique de la came 290, et il est donc possible de prédéterminer un quelconque profil de force au moyen de cette came 290.

Selon une autre variante, on peut par exemple prévoir une force d'actionnement Fa constante pendant toute la durée de l'injection.

Avantageusement, après l'injection, et de préférence avant d'éjecter le module réservoir 100 vide, l'utilisateur réarme le module moteur 200 en vue d'une prochaine utilisation avec un autre module réservoir 100.

Pour ce faire, dans l'exemple des figures 13 à 22, l'utilisateur ré-enroule le fil ou la courroie 230 autour de la roue d'enroulement 240. Ceci ramène la tige de piston 210 vers sa position de départ, avant actionnement, et recharge le ressort d'injection 220. L'utilisateur fait donc tourner la roue d'enroulement 240 en sens inverse par rapport à la phase d'injection. Pour ce faire, la roue d'enroulement 240 comporte plusieurs dents 241, qui coopèrent avec l'organe de verrouillage 260 à la manière d'un cliquet anti-retour. Lorsque l'utilisateur fait tourner la roue d'enroulement 240 afin de ré-enrouler le fil 230, l'organe de verrouillage 260 s'escamote et se verrouille après le passage de chaque dent 241. Ainsi, un nombre de dents 241 plus élevé facilite le réarmement du module moteur 200 par l'utilisateur.

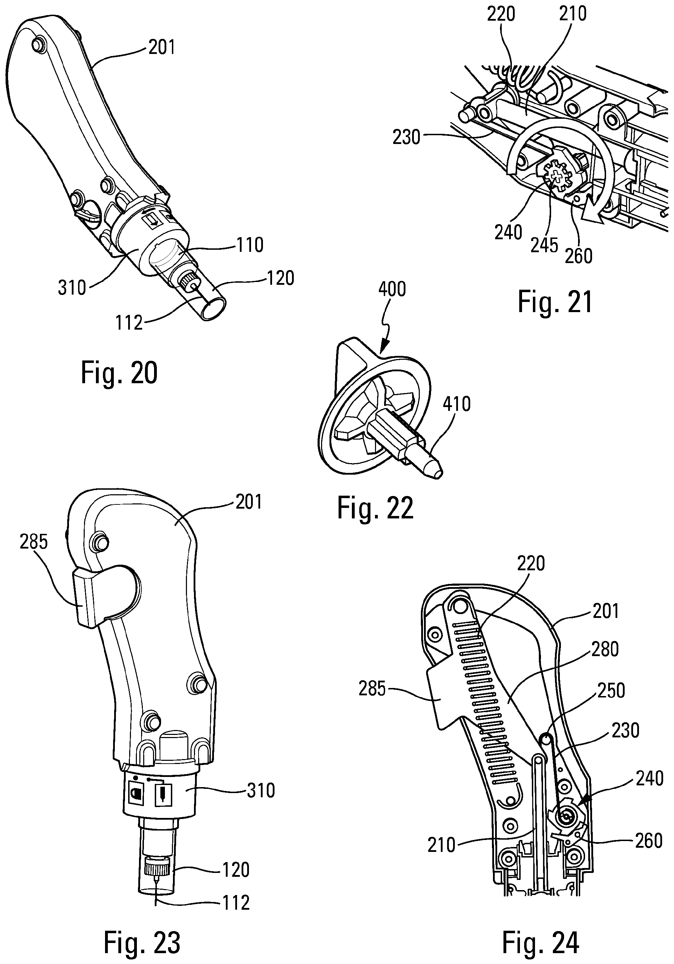

Avantageusement, une manivelle de réarmement 400 est prévue, dont un exemple est visible sur la figure 22. Cette manivelle 400 peut par exemple coopérer avec la roue d'enroulement 240 par l'intermédiaire de nervures 410 s'adaptant dans des rainures 245 correspondantes de la roue d'enroulement 240. En variante, on pourrait réaliser une forme de réarmement directement sur la roue d'enroulement 240, en remplacement de la manivelle. La manivelle 400 pourrait aussi être remplacée par une poignée comportant une denture adaptée à coopérer avec la roue d'enroulement 240.

Eventuellement, on peut prévoir un ensemble de pignons (non représentés) pour démultiplier la force d'enroulement et donc rendre celui-ci plus aisé pour l'utilisateur. Dans ce cas, on peut prévoir un pignon flottant coopérant d'une part avec la manivelle 400 et d'autre part avec une denture de la roue d'enroulement 240, ledit pignon flottant étant mobile dans un trou oblong du corps. Ceci permet au pignon flottant d'entrainer la roue d'enroulement 240 uniquement lorsqu'il tourne dans un seul sens de rotation, sans gêner la phase d'injection lorsque la roue d'enroulement 240 tourne dans l'autre sens.

Dans le mode de réalisation des figures 23 et 24, on prévoit de permettre à l'utilisateur d'agir directement sur l'effort d'injection, c'est-à-dire, d'augmenter manuellement l'effort d'injection. Ceci peut se faire en pressant directement sur le levier 280 via une partie 285 de celui-ci se projetant hors du corps 201 du module moteur 200. En variante, on pourrait prévoir une pièce de liaison accessible de l'extérieur du corps 201 et qui permettrait d'agir sur le levier 280.

La présente invention s'applique à des dispositifs utilisés notamment pour les traitements des maladies auto-immunes, par exemple du type arthrites rhumatoïdes, scléroses multiples, maladie de Crohn, pour les traitements contre le cancer, pour les traitements antiviraux, par exemple du type hépatites, pour les traitements contre le diabète, pour les traitements contre l'anémie ou pour les traitements de crises, par exemple en cas de choc anaphylactique.

Bien que la présente invention ait été décrite en référence à des modes de réalisation avantageux, il est entendu que diverses modifications sont possibles pour l'homme du métier sans sortir du cadre de la présente invention tel que défini par les revendications annexées.

## Revendications

1. Autoinjecteur comportant :
- un module réservoir (100) comportant un réservoir (110) contenant du produit fluide et un piston (111), et comportant une aiguille (112),
- un module moteur (200) comportant :
- un corps (201),
- une tige de piston (210) coopérant avec le piston (111) dudit réservoir (110), ladite tige de piston (210) étant déplacée entre une position de repos et une position d'injection dans laquelle ladite tige de piston (210) a déplacé le piston (111) du réservoir (110) pour injecter le produit fluide à travers ladite aiguille (112),
- un ressort d'injection (220) pour solliciter ladite tige de piston (210) vers sa position d'injection,
- des moyens de maitrise d'effort (280, 290), faisant varier de manière prédéterminée la force exercée par ledit ressort d'injection (220) sur ladite tige de piston (210) pendant toute la phase d'injection, lesdits moyens de maitrise d'effort comportant un levier (280) et une came (290), ledit levier (280) étant d'un côté monté pivotant sur ladite tige de piston (210), étant relié audit ressort d'injection (220), et coopérant de l'autre côté avec ladite came (290),
**caractérisé en ce que** l'autoinjecteur comporte un module interface (300) fixé au module moteur (200) et recevant un module réservoir (100) avant chaque actionnement, ledit module interface (300) comportant une bague de sélection (310) montée rotative sur le module moteur (200) et des éléments pivotants (320) pour fixer le module réservoir (100) dans le module interface (300), ladite bague de sélection (310) est mobile en rotation entre une position de verrouillage, dans laquelle l'insertion d'un module réservoir (100) est possible et l'actionnement du module moteur (200) est impossible, une position d'injection, dans laquelle l'actionnement du module moteur (200) est possible, une position de sécurité, dans laquelle un manchon actionneur (110) du module réservoir (100) revient en position déployée autour de ladite aiguille (112), et une position d'éjection, dans laquelle l'éjection du module réservoir utilisé est possible.

2. Autoinjecteur selon la revendication 1, dans lequel ledit module réservoir (100) comporte en outre un manchon actionneur (110) déplaçable par rapport audit réservoir (110) entre une position déployée dans laquelle il est disposé autour de ladite aiguille (112) et une position actionnée, dans laquelle il est déplacé axialement par rapport au réservoir (110) pour exposer l'aiguille (112).

3. Autoinjecteur selon la revendication 1 ou 2, dans lequel ledit module moteur (200) comporte une serrure qui bloque ladite tige de piston (210) en position de repos, ladite serrure comportant un fil (230) relié d'un côté à ladite tige de piston (210) et de l'autre côté à une roue d'enroulement dentée (240), un organe de verrouillage (260) coopérant avec une dent (241) de ladite roue d'enroulement dentée (240), ledit organe de verrouillage (260) étant mobile, notamment pivotant, entre une position de blocage, dans laquelle il empêche une rotation de la roue d'enroulement (240) dans le sens de la force d'actionnement (Fa) exercée par ledit ressort d'injection (220) sur ladite tige de piston (210), et une position d'actionnement, dans laquelle ladite rotation de la roue d'enroulement (240) est possible.

4. Autoinjecteur selon la revendication 3, dans lequel il est prévu une roue de renvoi (250) formant poulie pour le fil (230) entre ladite tige de piston (210) et ladite roue d'enroulement (240).

5. Autoinjecteur selon la revendication 3 ou 4, dans lequel ledit organe de verrouillage (260) comporte une extension (265), adaptée à coopérer avec un manchon actionneur (120) du module réservoir (100) lorsque ladite aiguille (112) a piqué le site à injecter, pour déplacer ledit organe de verrouillage (260) vers sa position d'actionnement.

6. Autoinjecteur selon l'une quelconque des revendications 3 à 5, comportant des moyens de réarmement (400), tel qu'une manivelle, pour ré-enrouler ledit fil (230) sur ladite roue d'enroulement (240), par rotation de ladite roue d'enroulement (240) dans le sens inverse à celui de la phase d'injection.

7. Autoinjecteur selon l'une quelconque des revendications précédentes, dans lequel ledit ressort d'injection (220) est fixé d'un côté audit corps (201) et de l'autre côté audit levier (280), une extrémité dudit levier étant pivotante sur ladite tige de piston (210) et l'autre extrémité se déplaçant le long de ladite came (290).

8. Autoinjecteur selon l'une quelconque des revendications précédentes, dans lequel ledit levier (280) effectue à la fois une rotation autour d'un point de ladite tige de piston (210) et un mouvement de translation par rapport audit corps (201), ces deux mouvements étant liés et réalisés dans un même plan.

## Patentansprüche

1. Autoinjektor aufweisend:
- ein Vorratsbehältermodul (100) mit einem Vorratsbehälter (110), der Fluidprodukt und einen Kolben (111) enthält, und einer Nadel (112),
- ein Motormodul (200), das Folgendes aufweist:
- einen Körper (201),
- einen Kolbenschaft (210), der mit dem Kolben (111) des Vorratsbehälters (110) zusammenwirkt, wobei der Kolbenschaft (210) zwischen einer Ruheposition und einer Injektionsposition bewegt wird, in der der Kolbenschaft (210) den Kolben (111) des Vorratsbehälters (110) bewegt hat, um das Fluidprodukt durch die Nadel (112) zu injizieren,
- eine Injektionsfeder (220) zum Vorspannen des Kolbenschafts (210) zu seiner Injektionsposition,
- Mittel zur Kraftbeherrschung (280, 290), die die Kraft, die durch die Injektionsfeder (220) auf den Kolbenschaft (210) ausgeübt wird, während der gesamten Injektionsphase auf vorbestimmte Weise variieren lassen, wobei die Mittel zur Kraftbeherrschung einen Hebel (280) und einen Nocken (290) umfassen, wobei der Hebel (280) auf einer Seite auf dem Kolbenschaft (210), der mit der Injektionsfeder (220) verbunden ist, schwenkbar angebracht ist, und auf der anderen Seite mit dem Nocken (290) zusammenwirkt, **dadurch gekennzeichnet, dass** der Autoinjektor ein Schnittstellenmodul (300) aufweist, das am Motormodul (200) befestigt ist und vor jeder Betätigung ein Vorratsbehältermodul (100) aufnimmt, wobei das Schnittstellenmodul (300) einen Auswahlring (310), der drehbar auf dem Motormodul (200) angebracht ist, und Schwenkelemente (320) zur Befestigung des Vorratsbehältermoduls (100) im Schnittstellenmodul (300) aufweist, der Auswahlring (310) zwischen einer Verriegelungsposition, in der das Einsetzen eines Vorratsbehältermoduls (100) möglich und die Betätigung des Motormoduls (200) unmöglich ist, einer Injektionsposition, in der die Betätigung des Motormoduls (200) möglich ist, einer Sicherheitposition, in der eine Betätigungshülse (110) des Vorratsbehältermoduls (100) in die ausgefahrene Position um die Nadel (112) herum zurückkehrt, und einer Auswurfposition, in der das Auswerfen des benutzten Vorratsbehältermoduls möglich ist, drehbeweglich ist.

2. Autoinjektor nach Anspruch 1, wobei das Vorratsbehältermodul (100) ferner eine Betätigungshülse (110) aufweist, die bezogen auf den Vorratsbehälter (110) zwischen einer ausgefahrenen Position, in der sie um die Nadel (112) herum angeordnet ist, und einer betätigten Position verschiebbar ist, in der sie bezogen auf den Vorratsbehälter (110) axial verschoben ist, um die Nadel (112) freizulegen.

3. Autoinjektor nach Anspruch 1 oder 2, wobei das Motormodul (200) ein Schloss aufweist, das den Kolbenschaft (210) in der Ruheposition blockiert, wobei das Schloss einen Faden (230) aufweist, der auf einer Seite mit dem Kolbenschaft (210) und auf der anderen Seite mit einem gezahnten Wickelrad (240) verbunden ist, wobei ein Verriegelungsorgan (260) mit einem Zahn (241) des gezahnten Wickelrads (240) zusammenwirkt, wobei das Verriegelungsorgan (260) zwischen einer Blockageposition, in der es eine Drehung des Wickelrads (240) in Richtung der Betätigungskraft (Fa) verhindert, die von der Injektionsfeder (220) auf den Kolbenschaft (210) ausgeübt wird, und einer Betätigungsposition, in der die Drehung des Wickelrads (240) möglich ist, beweglich, insbesondere schwenkbar ist.

4. Autoinjektor nach Anspruch 3, wobei ein Umlenkrad (250), das eine Laufrolle für den Faden (230) bildet, zwischen dem Kolbenschaft (210) und dem Wickelrad (240) vorgesehen ist.

5. Autoinjektor nach Anspruch 3 oder 4, wobei das Verriegelungsorgan (260) eine Verlängerung (265) aufweist, die geeignet ist, mit einer Betätigungshülse (120) des Vorratsbehältermoduls (100) zusammenzuwirken, wenn die Nadel (112) in die Injektionsstelle eingestochen hat, um das Verriegelungsorgan (260) zu seiner Betätigungsposition zu bewegen.

6. Autoinjektor nach einem der Ansprüche 3 bis 5, aufweisend Rückstellmittel (400) wie etwa eine Kurbel zum Wiederaufwickeln des Fadens (230) auf das Wickelrad (240) durch Drehung des Wickelrads (240) in die Richtung, die jener der Injektionsphase entgegengesetzt ist.

7. Autoinjektor nach einem der vorhergehenden Ansprüche, wobei die Injektionsfeder (220) auf einer Seite am Körper (201) und auf der anderen Seite am Hebel (280) befestigt ist, wobei ein Ende des Hebels auf dem Kolbenschaft (210) schwenkbar ist und sich das andere Ende den Nocken (290) entlang bewegt.

8. Autoinjektor nach einem der vorhergehenden Ansprüche, wobei der Hebel (280) sowohl eine Drehung um einen Punkt des Kolbenschafts (210) und eine Translationsbewegung bezogen auf den Körper (201) ausführt, wobei diese zwei Bewegungen verbunden sind und in ein und derselben Ebene ausgeführt werden.

## Claims

1. An autoinjector, comprising:
- a reservoir module (100) comprising a reservoir (110) containing fluid and a piston (111), and including a needle (112),
- a driver module (200) comprising:
- a body (201),
- a piston rod (210) that co-operates with the piston (111) of said reservoir (110), said piston rod (210) being moved between a rest position and an injection position in which said piston rod (210) has moved the piston (111) of the reservoir (110) so as to inject the fluid through said needle (112),
- an injection spring (220) for urging said piston rod (210) towards its injection position,
- force control means (280, 290), for causing the force exerted by said injection spring (220) on said piston rod (210) to vary in predetermined manner throughout the injection stage, said force control means comprising a lever (280) and a cam (290), said lever (280) being pivotally mounted at one end on said piston rod (210), being connected to said injection spring (220), and co-operating at its other end with said cam (290),
**characterized in that** it comprises an interface module (300) that is fastened to the driver module (200) and that receives a reservoir module (100) before each actuation, said interface module (300) comprising a selector ring (310) that is mounted to turn on the driver module (200), and pivot elements (320) for fastening the reservoir module (100) in the interface module (300), said selector ring (310) is movable in turning between a locked position in which insertion of a reservoir module (100) is possible and actuation of the driver module (200) is not possible, an injection position in which actuation of the driver module (200) is possible, a safety position in which an actuator sleeve (110) of the reservoir module (100) returns into its deployed position around said needle (112), and an ejection position in which ejection of the used reservoir module is possible.

2. An autoinjector according to claim 1, wherein said reservoir module (100) further comprises an actuator sleeve (110) that is movable relative to said reservoir (110) between a deployed position in which it is arranged around said needle (112), and an actuated position in which it is moved axially relative to the reservoir (110) so as to expose the needle (112).

3. An autoinjector according to claim 1 or claim 2, wherein said driver module (200) includes a lock that blocks said piston rod (210) in its rest position, said lock comprising a thread (230) that is connected at one end to said piston rod (210) and at its other end to a toothed winder wheel (240), a locking member (260) co-operating with a tooth (241) of said toothed winder wheel (240), said locking member (260) being movable, in particular pivotable, between a blocking position in which it prevents the winder wheel (240) from turning in the direction of the actuation force (Fa) exerted by said injection spring (220) on said piston rod (210), and an actuated position in which said turning of the winder wheel (240) is possible.

4. An autoinjector according to claim 3, wherein a deflector wheel (250) forming a pulley for the thread (230) is provided between said piston rod (210) and said winder wheel (240).

5. An autoinjector according to claim 3 or claim 4, wherein said locking member (260) includes an extension (265) that is adapted to co-operate with an actuator sleeve (120) of the reservoir module (100) when said needle (112) has jabbed the site that is to be injected, so as to move said locking member (260) towards its actuated position.

6. An autoinjector according to any one of claims 3 to 5, including recocking means (400), such as a knob, for rewinding said thread (230) on said winder wheel (240) by turning said winder wheel (240) in the opposite direction to its direction during the injection stage.

7. An autoinjector according to any preceding claim, wherein said injection spring (220) is fastened at one end to said body (201) and at its other end to said lever (280), one end of said lever being pivotable about said piston rod (210) and its other end being movable along said cam (290).

8. An autoinjector according to any preceding claim, wherein said lever (280) performs both a pivoting movement about a point of said piston rod (210) and also a movement in translation relative to said body (201), these two movements being linked and performed in a single plane.
